# EUROPEAN PATENT SPECIFICATION

(11) **EP 0 380 649 B1**
(45) Date of publication and mention of the grant of the patent: **16.10.1996**
(21) Application number: 89909301.7
(22) Date of filing: 02.08.1989
(51) Int. Cl.: A01N 63/02, C12N 1/20

(54) **BIOLOGICAL INOCULANT EFFECTIVE AGAINST APHANOMYCES**
BIOLOGISCHER IMPFSTOFF, WIRKSAM GEGEN APHANOMYCES
INOCULANT BIOLOGIQUE EFFICACE CONTRE APHANOMYCES

(30) Priority: 03.08.1988 US 277810
(43) Date of publication of application: 08.08.1990
(73) Proprietor: WISCONSIN ALUMNI RESEARCH FOUNDATION, Madison, WI 53707-7365 (US)
(72) Inventor: PARKE, Jennifer, L., Madison, WI 53711 (US)
(74) Representative: Ellis-Jones, Patrick George Armine
(86) International application number: US8903277
(87) International publication number: WO9001327

(56) References cited:
- WO-A-88/00966
- CA-A- 1 172 585
- JP-A-59 062 509
- US-A- 3 819 829
- US-A- 4 588 584
- US-A- 4 798 723
- PHYTOPATHOLOGY, vol. 77, no. 12, 1987; J.L. PARKE, p. 1688
- CHEMICAL ABSTRACTS, vol. 87, no. 3, 18 July 1977, Columbus, OH (US); N.N. KIRIK et al., p. 146, no. 17075x
- BIOLOGICAL CONTROL OF PLANT PATHOGENS, Kelma (ed.), 1982; BAKER, p. 161
- BIOLOGICAL CULTURAL TESTS, vol. 3, 1985; CAVILEER, p. 79
- KOREAN JOURNAL OF PLANT PATHOLOGY, vol. 3, 1987; CHO, pp. 313-317
- CANADIAN JOURNAL OF MICROBIOLOGY, vol. 31, 1985; GAGNE, pp. 856-860
- ANNALS OF THE PHYTOPATHOLOGICAL SOCIETY OF JAPAN, vol. 52, 1986; LEE, pp. 175-183

## Description

### Field of the Invention

The present invention is generally directed to inoculants for plants, and particularly directed to a biological inoculant effective in controlling root rot of plants, such as peas, caused by the fungus Aphanomyces euteiches.

### Background of the Invention

Farm crops are continually plagued by a variety of pests which can stunt or damage crop growth or even completely destroy the crop. Some of the pests are in the form of weeds which grow similarly to the desired plant and compete for the nutrients provided by soil and water. Other pests are in the form of pathogens such as fungi and bacteria which are found in association with many plants.

One of the more serious problems associated with fungal pathogens in plants is root rot. For example, pea root rot caused by the fungus Aphanomyces euteiches is a serious problem in pea-growing areas, particularly in Wisconsin and other Great Lake states. The Aphanomyces fungus infects not only peas, but also snap beans and alfalfa, accounting for 10 to 15% losses in yield. In extreme cases, some fields, where the fungus population has been built up over the period of several years, have become essentially useless for these crops.

Despite efforts to develop fungicides and commercially acceptable pea cultivars with resistance to this pathogen, there is presently no commercially available product capable of controlling Aphanomyces. Currently, the best way to avoid the disease loss is to avoid planting susceptible crops in soils with a high population of the Aphanomyces fungus. Unfortunately, the fungus can survive for many years in field soil and a long rotational time to other crops is not practical. As a result, there is a need to find an alternative disease control strategy to eliminate root rot caused by Aphanomyces and possibly other fungi.

There is increasing interest in the use of living organisms to control such diseases. Microscopic organisms are present in soil in populations of approximately 1 billion per cubic inch of soil. Some of the microorganisms cause disease and some are beneficial. The beneficial microorganisms are of major interest. It has long been known in agriculture that certain of these microbial inoculants can be used to facilitate the growth of certain plant species or to assist the plants in suppressing particular pathogenic organisms. For example, it has been a common practice to inoculate soybeans and other legumes at planting with bacterial cultures of the genus Rhizobium so that nitrogen-fixing nodules will form as a result of the plant-bacterium symbiosis.

Reference is now made to U.S. Patent No. 4,588,584 to Lumsden, et al. which discloses a particular species of Pseudomonas cepacia which is effective in controlling Pythium diseases of cucumber and peas. There is also much literature on the use of Pseudomonas fluorescens as a biocontrol agent against various plant diseases, but not against the fungus Aphanomyces. The term "biocontrol agent", as used herein, refers to a living organism which controls diseases.

In addition strains for the biological control of Phytophera have been disclosed (Cho, Korean J. Plant Pathol., 1987, 313) and for control of Aphanomyces (Parke, Phytopathology (1987), Vol. 77, No. 12). Studies on the seed bacterization of sugar beet infected with Aphanomyces have also been reported (Lee et al., Ann. Phytopath. Soc., Japan, Vol 52, pp 175-183, 1986).

### Summary of the Invention

It is therefore an object of the invention to provide a biocontrol agent which is effective in biologically controlling pea root rot in the field.

It is also an object of the present invention to provide a biocontrol agent which is effective in reducing plant mortality in peas and other vegetable and field crops.

It is further an object of the present invention to provide a process for increasing the crop yield in Aphanomyces-infested soils.

The present invention provides a process for controlling *Aphanomyces* fungal disease, especially *Aphanomyces euteiches* fungal disease, of plants comprising inoculating the plants with biologically pure culture of a bacterial strain selected from the group consisting of *Pseudomonas cepacia* AMMA (ATCC Accession No. 53796), *Pseudomonas cepacia* AMMD (ATCC Accession No. 53795), *Pseudomonas fluorescens,* (ATCC Accession No. 53794), *Corynebacterium flaccufaciens* 5A (ATCC Accession No. 53934), *Bacillus* strain AM (ATCC Accession No. 53933) and *Bacillus* strain CRK419 (ATCC Accession No. 53935), and mixtures thereof.

The process of the present invention may provide increased seed germination, decreased plant mortality and increased yield of a pea plant by promoting growth.

The present invention is also directed to a biological inoculant suitable for controlling *Aphanomyces* fungal diseases on plants comprising a biologically pure culture of a bacteria selected from the group consisting of strains *Pseudomonas cepacia* AMMA and AMMD, *Pseudomonas fluorescens* PRA25, C*orynebacterium flaccufaciens* 5A, *Bacillus* strain AM, and CRK419, and mixtures thereof.

The present invention is also directed to an agriculturally useful composition comprising a pea seed inoculated with an inoculant of either *Pseudomonas cepacia* AMMA or AMMD or *Pseudomonas fluorescen*s PRA25 suitable for controlling *Aphanomyces* fungal disease.

The invention further provides biologically pure cultures of *Pseudomonas cepacia* AMMA or AMMD, *Pseudomonas fluorescens* PRA25, *Corynebacterium flaccufacien*s 5A, *Bacillus* strain AM or *Bacillus* strain CRK419 suitable for use in the control of *Aphanomyces* fungal disease of plants.

The inoculum which controls Aphanomyces on field crops, such as peas, is also disclosed in this invention. As used herein, the term "inoculum" means a biological control agent which is introduced onto a host substance or into soil. The inoculum comprises an essentially biologically pure culture of the bacteria mentioned in the previous paragraphs.

The bacterial strains and their process of use, disclosed in the present invention, represent a significant advance in controlling Aphanomyces. Because the bacterial strains are a biologically pure culture of a natural biological organism, massive quantities of the inoculum can be applied to the Aphanomyces infested area with little danger of environmental contamination. In view of public concern for ground water contamination and aerial pollution from pesticides, the form of control disclosed in the present invention is an attractive and economic alternative to chemical pesticides and other methods of control.

Other objects, advantages and features of the present invention will become apparent from the following specification when taken in conjunction with the accompanying drawing.

### Brief Description of the Drawings

Figure 1 is a graph illustrating the results of the mass spectrometric analysis of the fatty acid profile of Pseudomonas cepacia AMMA;
Figure 2 is a graph illustrating the results of the mass spectrometric analysis of the fatty acid profile of Pseudomonas cepacia AMMD;
Figure 3 is a graph illustrating the results of the mass spectrometric analysis of the fatty acid profile of Pseudomonas fluorescens PRA25: and
Figure 4 illustrates a schematic view of a plant bioassay useful for observing biocontrol activity.
Figure 5 is a graph illustrating the results of the mass spectrometric analysis of the fatty acid profile of Corynebacterium flaccumfaciens 5A.
Figure 6 is a graph illustrating the results of the mass spectrometric analysis of the fatty acid profile of bacterial strain AM.
Figure 7 is a graph illustrating the results of the mass spectrometric analysis of the fatty acid profile of bacterial strain CRK419.

### Detailed Description of the Invention

The present invention is directed to improving the growth and survival rate of field crops infested with the fungus Aphanomyces, and particularly the strain Aphanomyces euteiches, by inoculating the field crop with a biologically pure bacterial inoculant of the selected strains of the species Pseudomonas cepaci, Pseudomonas fluorescens, Corynebacterium flaccumfaciens, and a strain of Bacillus. The particular strains of the bacteria involved in the present invention were discovered by the inventor and are identified by the following nomenclature, as presently known:
Pseudomonas cepacia AMMA
Pseudomonas cepacia AMMD
Pseudomonas fluorescens PRA25
Corynebacterium flaccumfaciens 5A
Bacillus/Corynebacterium sp AM
Bacillus CRK419

The above-referenced bacterial strains were initially isolated from over 200 strains of bacteria associated with pea plants in the field. The bacterial strains Pseudomonas fluorescens PRA25 and strains 5A and AM were initially isolated from the rhizosphere of healthy appearing pea plants grown in soil at the University of Wisconsin - Arlington Experimental Farms Pea Root Rot Nursery, a naturally infested pea root rot area. The bacterial strains Pseudomonas cepacia AMMA and Pseudomonas cepacia AMMD were initially isolated from the rhizosphere of healthy appearing pea plants grown in containment in soil known to be infested with the Aphanomyces fungus from the University of Wisconsin-Arlington Experimental Farm. The strain of uncertain taxonomy (probably Bacillus) designated CRK419 was isolated from a corn field in which peas were previously cultivated near Fredonia, Ozaukee County, Wisconsin.

The targeted bacterial strains were collected in the following manner. The root system from the pea plants was removed and agitated to shake off the excess soil. The hypocotyl and epicotyl segments of the roots were placed in distilled water, sonicated, and thereafter isolated in a plate dilution process, known to the art, in a TSAC (tryptic soy agar cyclohexamide) medium. Cyclohexamide is an anti-fungal agent. Colonies were thereafter selected and screened according to methods known to the art. The bacterial strains were thereafter stored in a DMSO solution at approximately -80°C until required for use.

It has been found that the bacterial strains may be mass produced in culture with relative ease. The strains are cultured in a suitable culture medium such as a commercially available nutrient broth yeast (NBY) extract. As the bacterial strains grow and multiply, essentially biologically pure cultures of the strains are formed which may be collected. The term "biologically pure culture" is used herein to refer to cultures of bacteria that have essentially no concentration of other strains of bacteria.

The bacteria strains were then screened for biocontrol activity in soil which has been naturally infested or artificially infested with the Aphanomyces fungus in order to determine which bacterial strains are effective biocontrol agents.

The selected biocontrol agents were coated onto the plant seeds, e.g., the pea seeds, prior to planting. A preferred method for coating the seeds is to combine the bacterial strain with a biologically non-interfering liquid carrier for application onto the seeds. A carrier shall be deemed "biologically non-interfering" if it does not prevent the bacterial strains from growing, and if it does not affect Aphanomyces in the absence of the bacteria. The nutrient medium in which the bacteria were cultured has been found to be a satisfactory medium. A suitable fungicide, i.e., captan, may also be coated on the seeds. The cultures survive air-drying after seed coating. The preferred carrier is a water-based liquid, preferably sterile distilled water.

Although coating the seed with the bacterial strain is preferred, other processes which provide a convenient means for distributing the bacterial strain to the Aphanomyces fungi fall within the scope and spirit of the invention. For example, the bacterial strain may be directly applied to the soil prior to planting the seeds.

Whether the inoculant is coated actually on the plant seed or inserted into the furrows into which the seeds are planted, the inoculant is preferably diluted with a suitable carrier or extender so as to make the inoculant easier to handle and to provide a sufficient quantity of material so as to be capable of easy human handling. Examples of suitable carriers include water, granular minerals, such as vermiculite, soils or peat.

To enable others to obtain a culture of these strains, samples have been deposited with the American Type Culture Collection (ATCC), being identified by the accession number and date of deposit as follows:

| Bacterial Strain | ATCC Accession No. | Date of Deposit |
|---|---|---|
| Pseudomonas cepacia AMMA | 53796 | 7/22/88 |
| Pseudomonas cepacia AMMD | 53795 | 7/22/88 |
| Pseudomonas fluorescens PRA25 | 53794 | 7/22/88 |
| Corynebacterium flaccumfaciens 5A | 53934 | 7/26/89 |
| Bacillus/Corynebacterium AM | 53933 | 7/26/89 |
| Bacillus CRK419 | 53935 | 7/26/89 |

To further identify the bacterial strains, a fatty acid profile for each rhizosphere culture was determined by mass spectrometric analysis. The term "rhizosphere", as used herein, refers to the zone of soil subject to the influence of the plant roots.

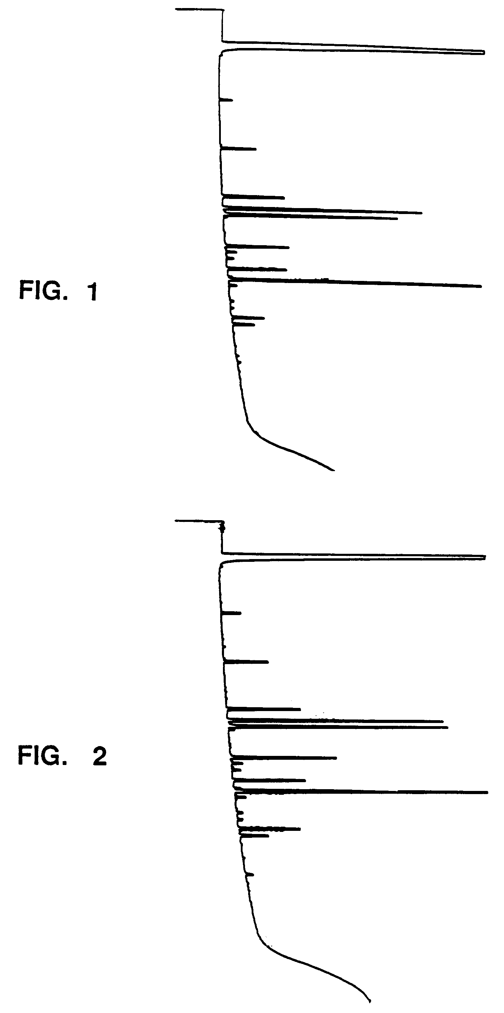

With reference to Figure 1, the results of the tests to determine the fatty acid profile for Pseudomonas cepacia AMMA are presented below in Table 1:

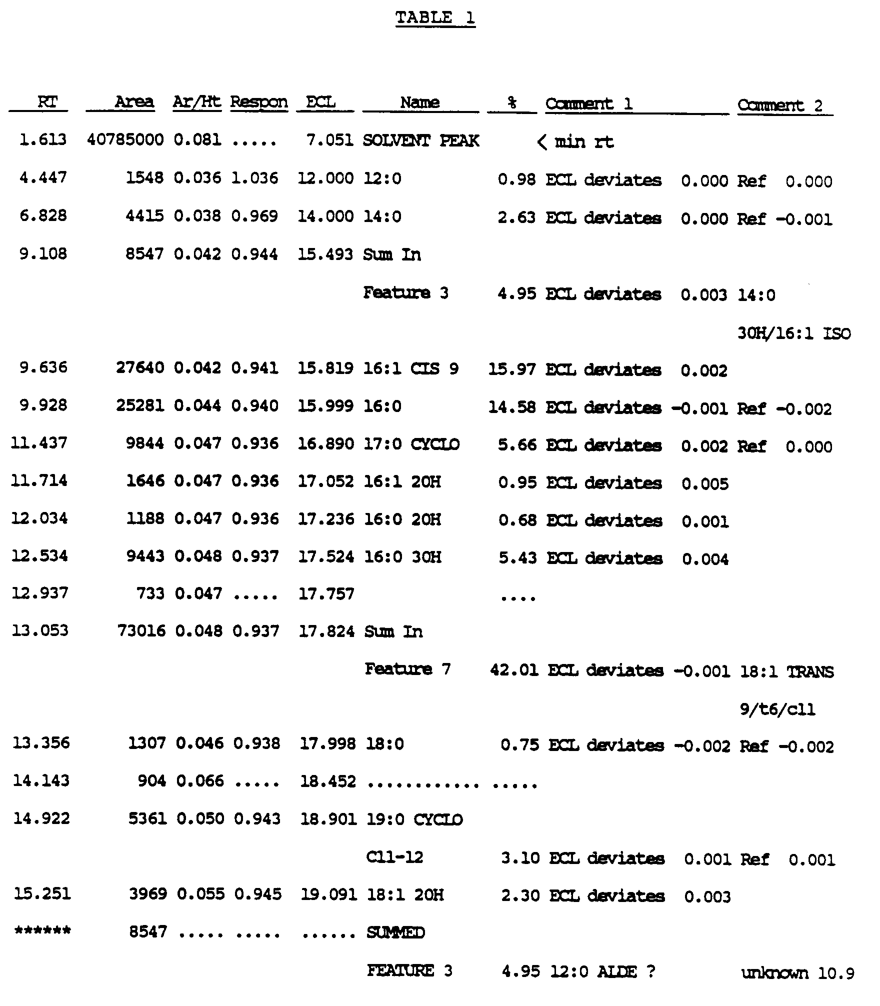

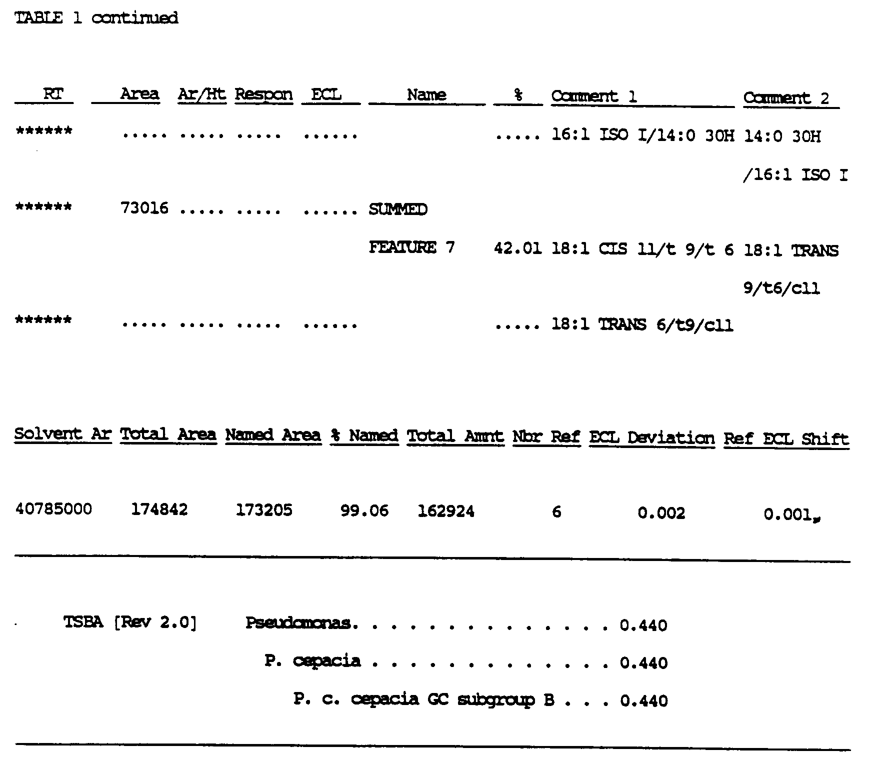

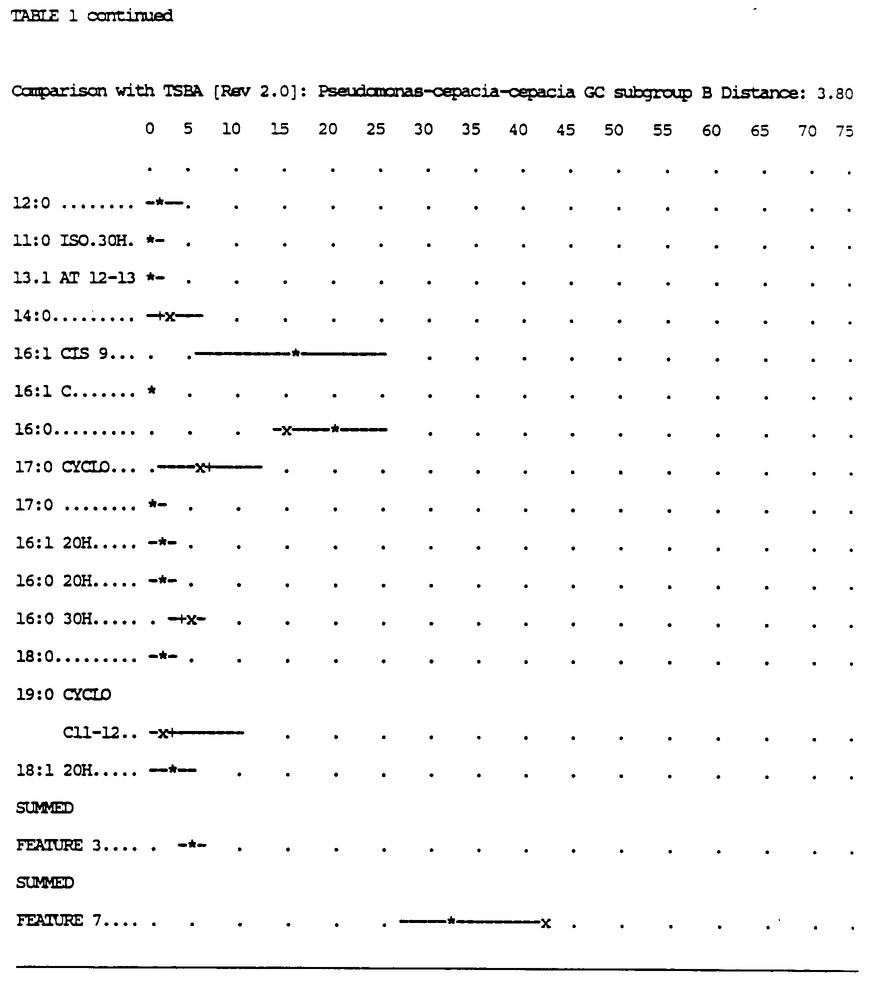

With reference to Figure 2, the results of the fatty acid profile for Pseudomonas cepacia AMMD are presented below in Table 2:

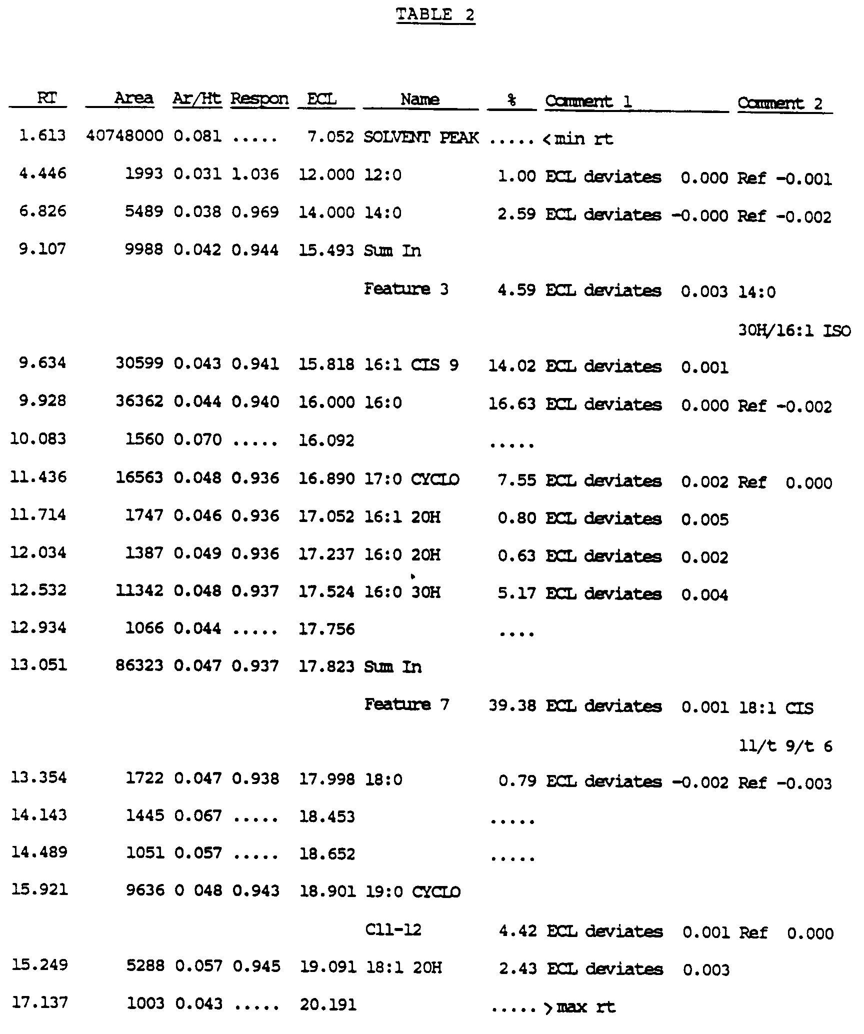

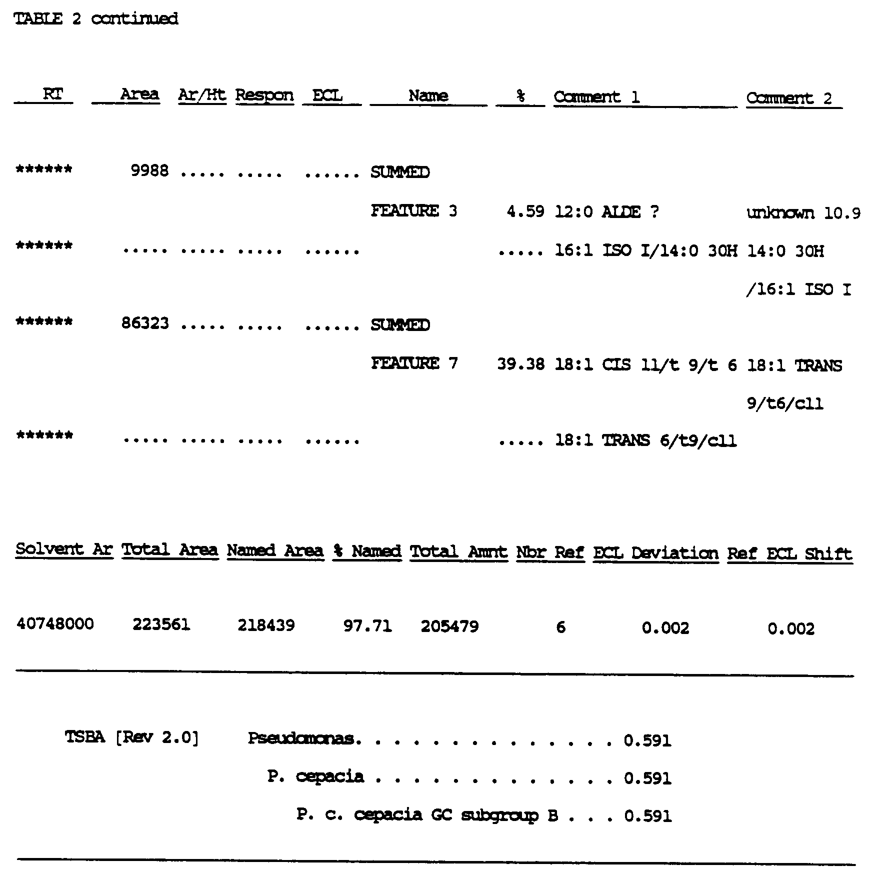

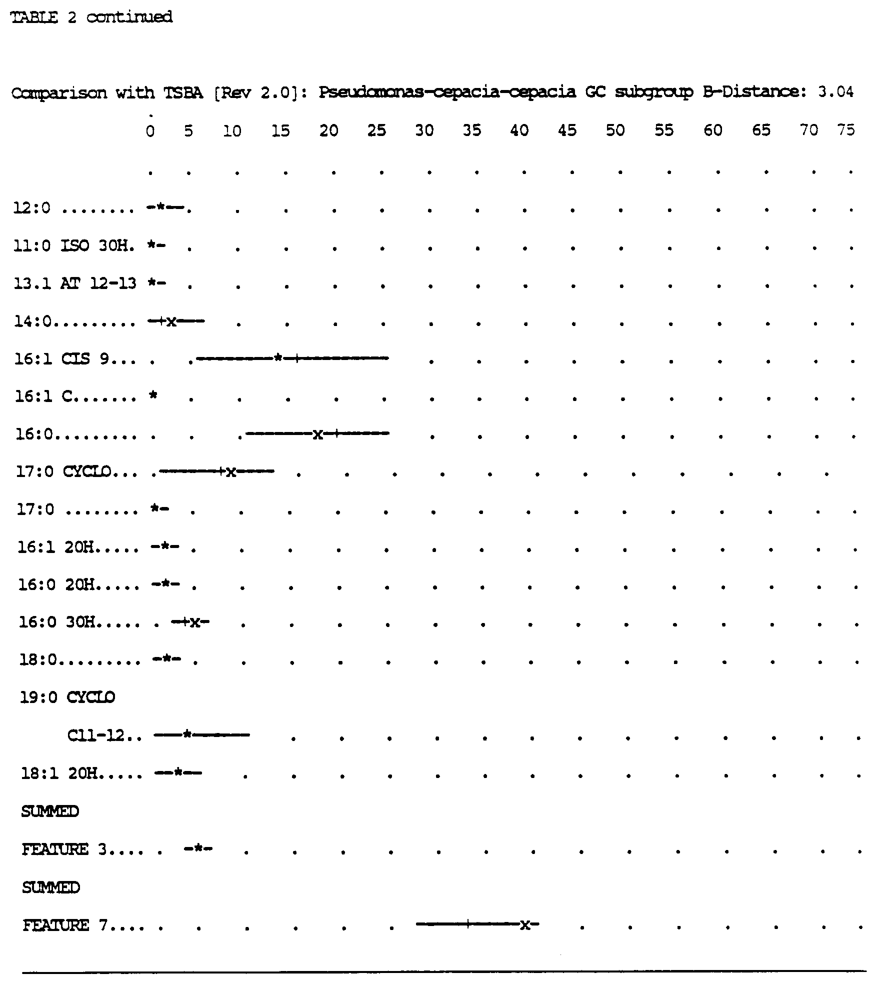

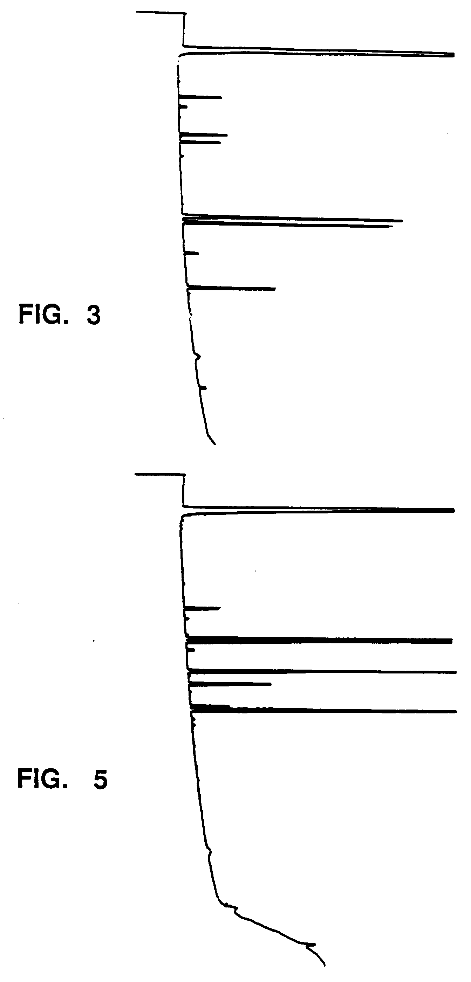

With reference to Figure 3, the results of the tests to determine the fatty acid profile for Pseudomonas fluorescens PRA25 are presented below in Table 3:

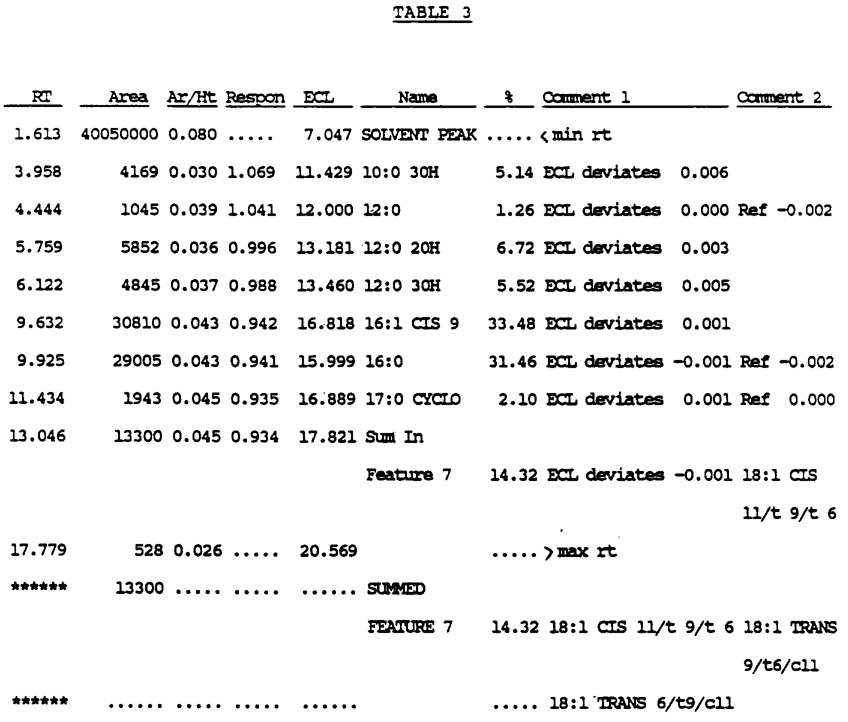

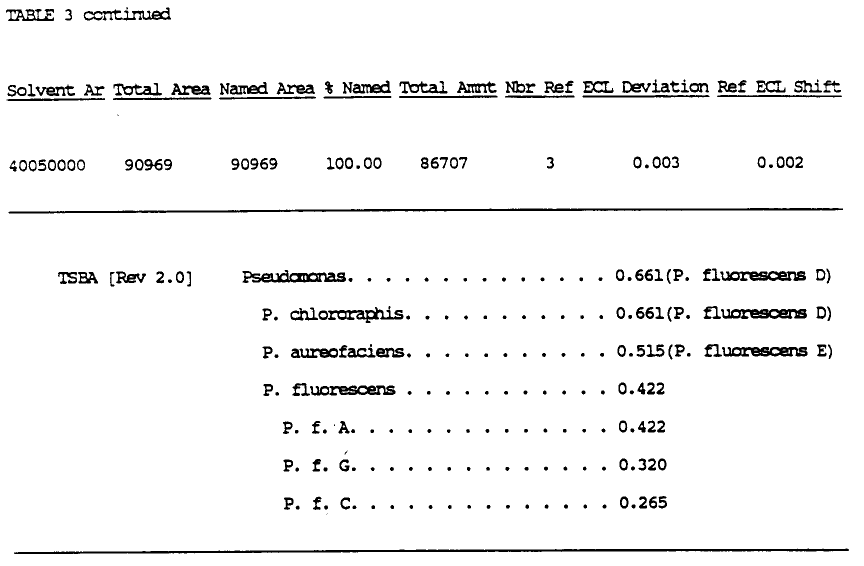

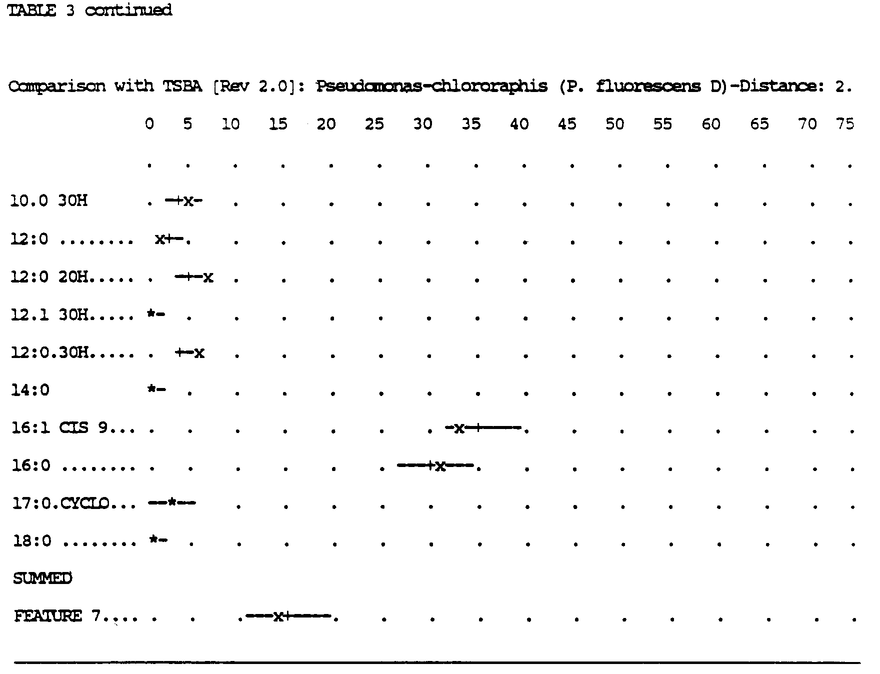

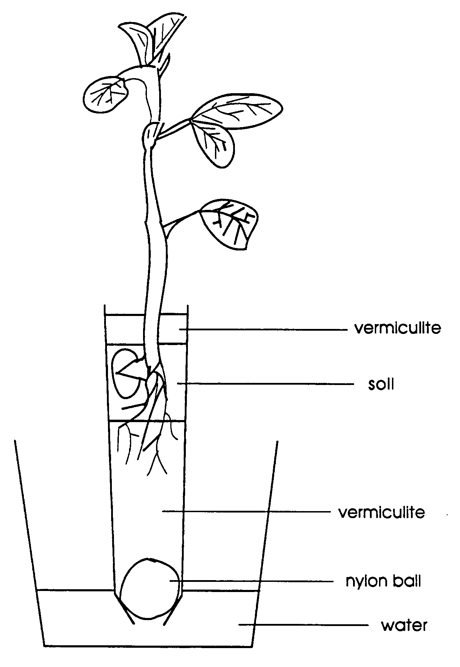

Fig. 5 illustrate: the fatty acid profile, determined by mess spectrometer for strain 5A. The exact taxonomical classification of strain 5A is not certain, although it is in the Corynebacterium or Bacillus groups, and it is currently believed that the organism is properly classified as Corynebacterium flaccumfaciens. It is a gram positive, non-motile rod and on NBY forms smooth bright yellow colonies, with margins entire. The bacteria are aerobic, catalase positive, oxidase negative and grow on TTC agar. To further firmly fix the species classification, it would be necessary to perform a thin layer chromatographic analysis of the whole organism methanolysates. The results of the fatty acid analysis are recapitulated in the following Table 4:

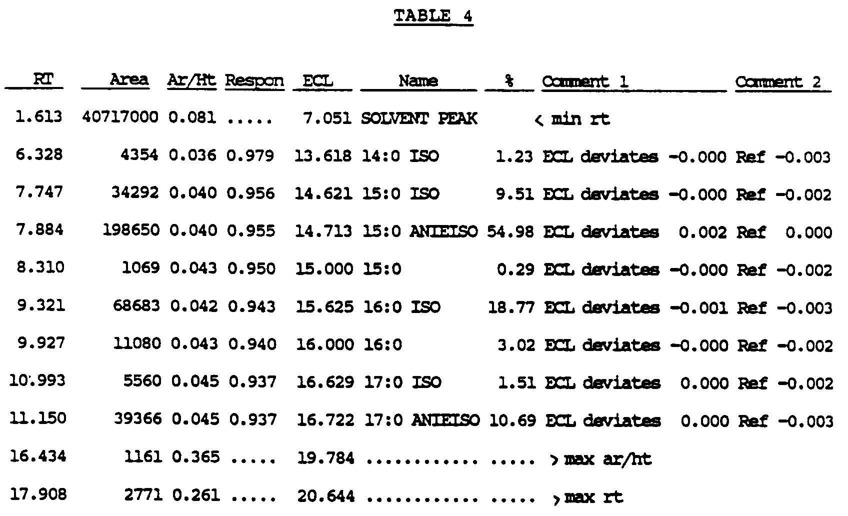

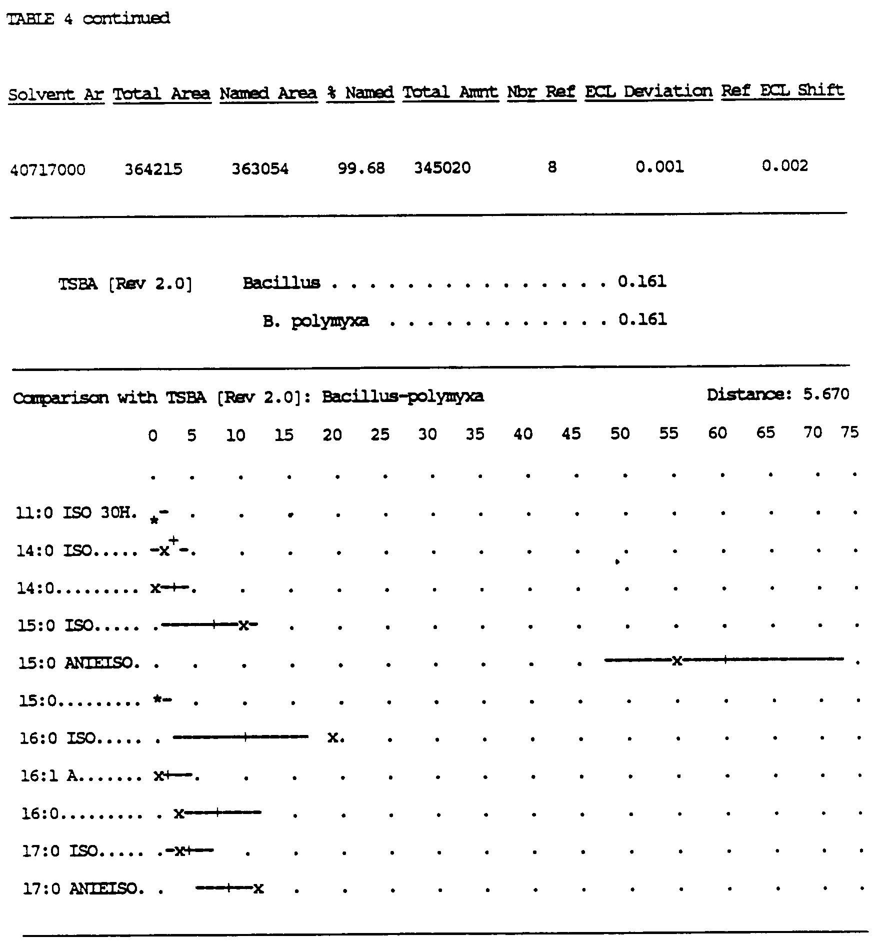

The strain AM is also not unequivocally classified. It appears to belong to the Bacillus polymyxa/circulans/macerans group. It may also, however, be Corynebacterium as well. With reference to Fig. 6, the results of the fatty acid profile for strain AM are presented below in Table 5:

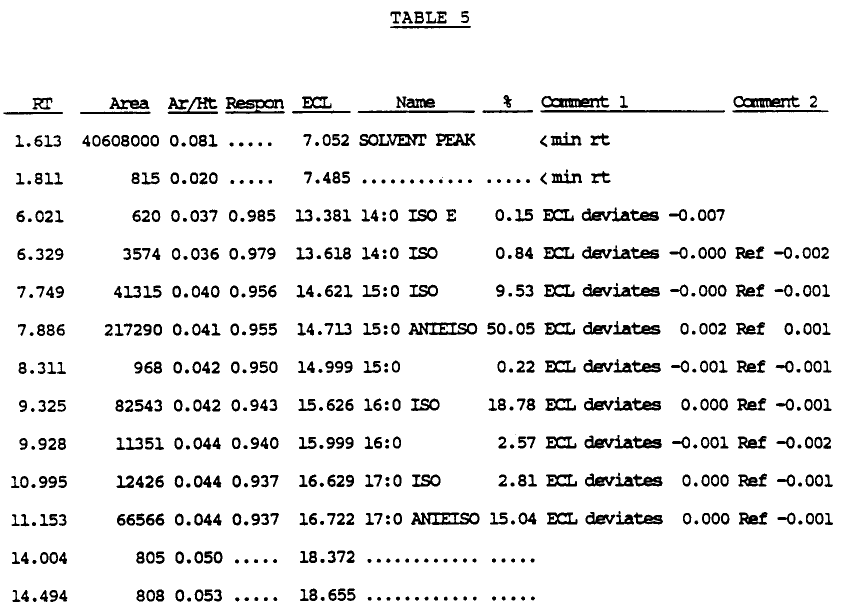

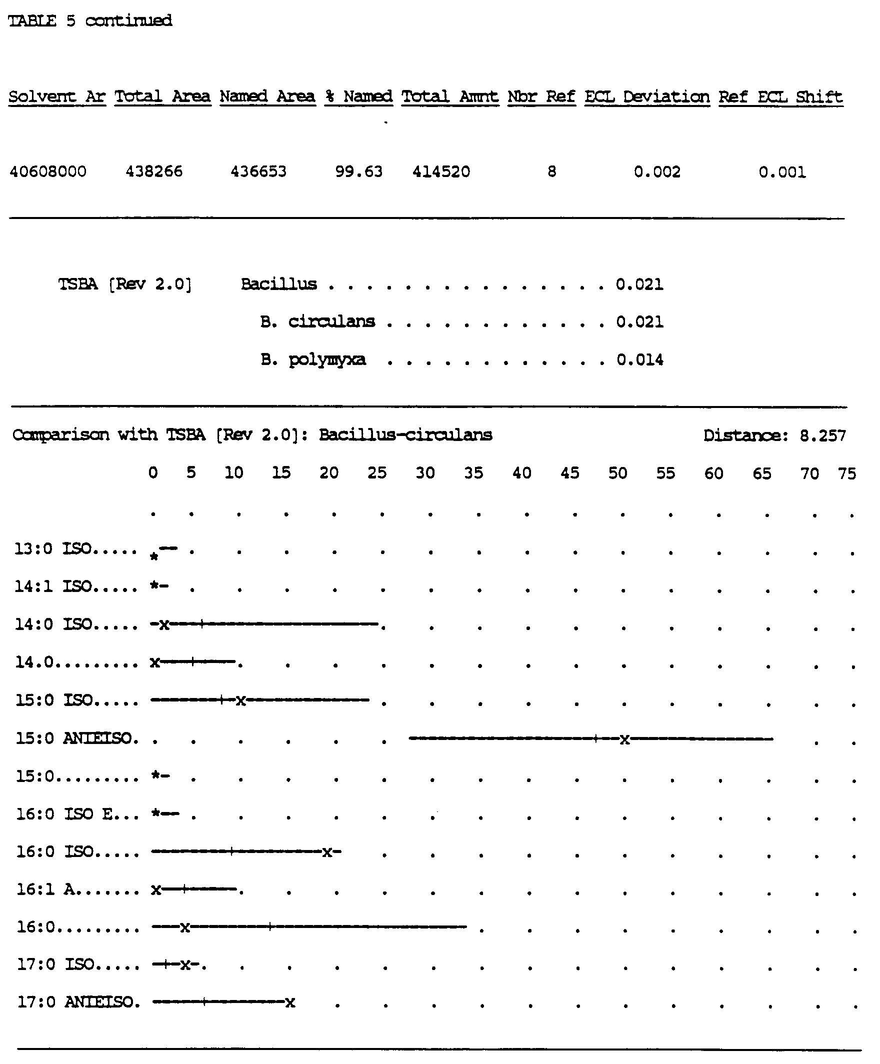

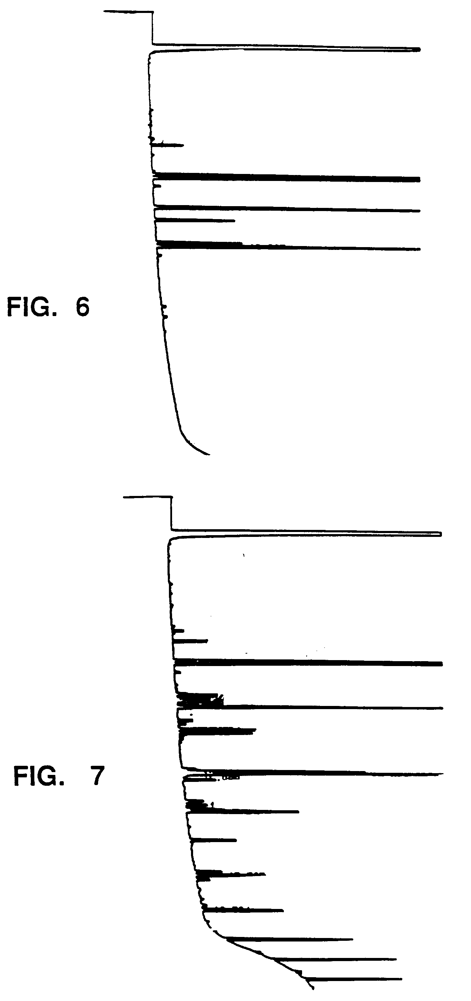

The strain CRK419 is a Bacillus strain, perhaps of Bacillus firmus. Referring now to Fig. 7, the results of the fatty acid profile of the Bacillus strain CRK419 is presented referring also to the following Table 6:

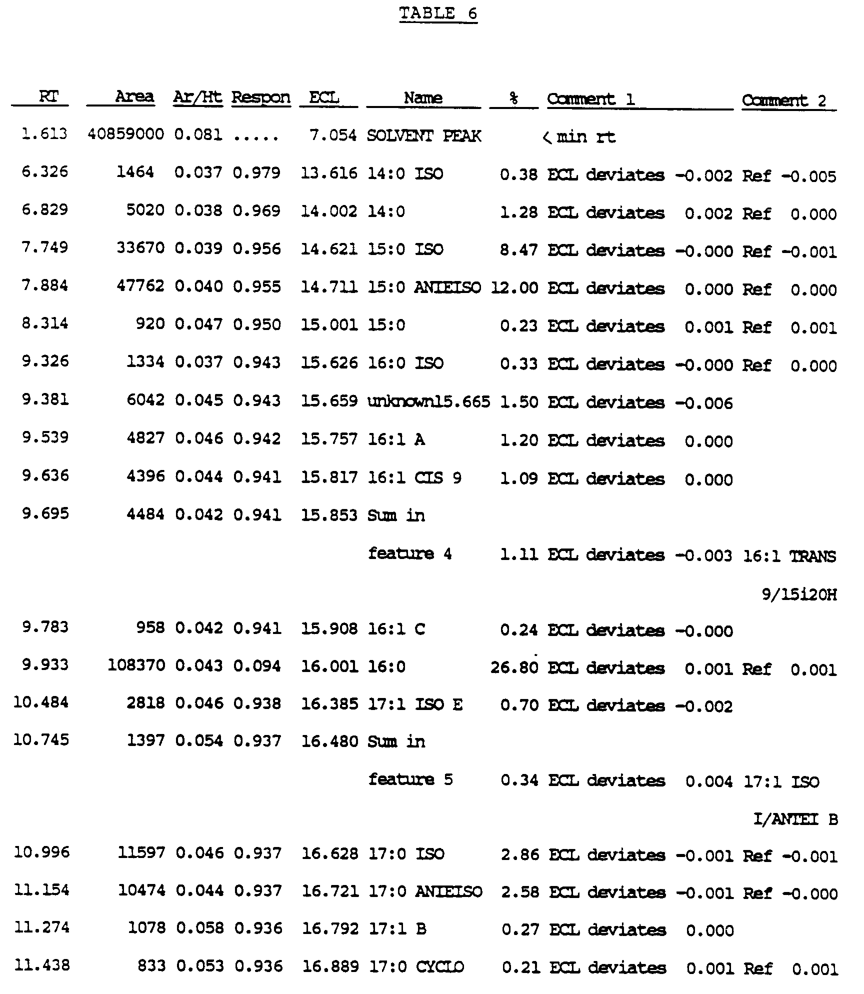

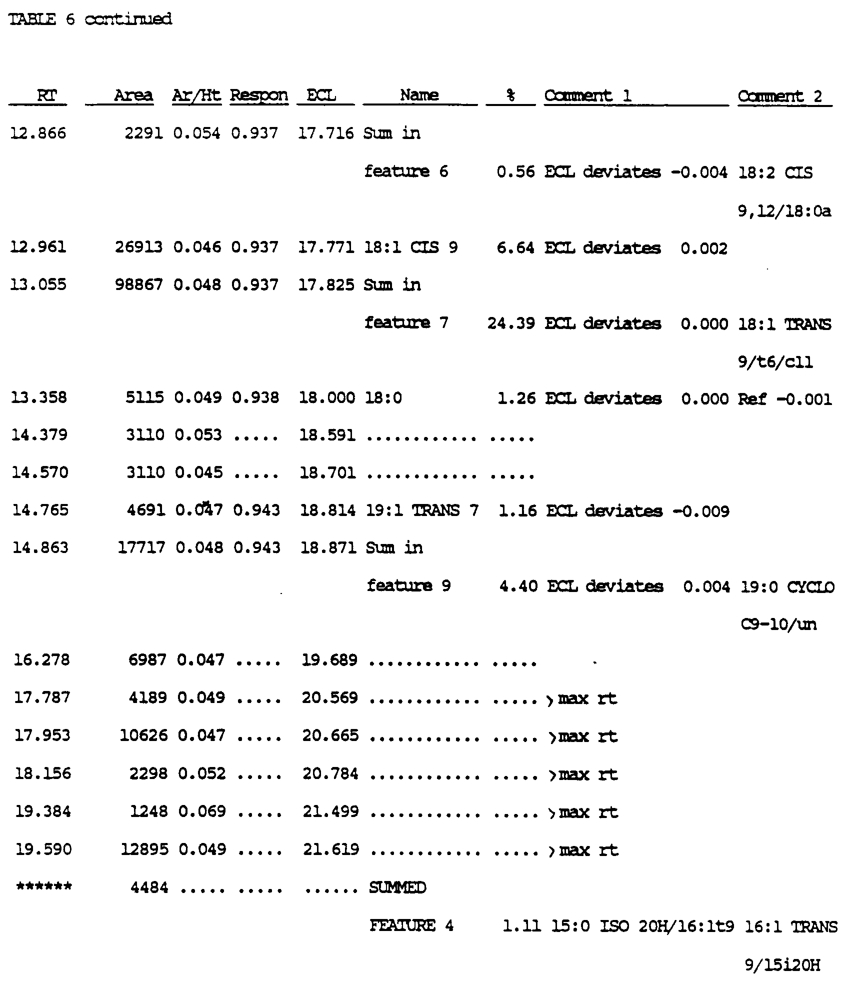

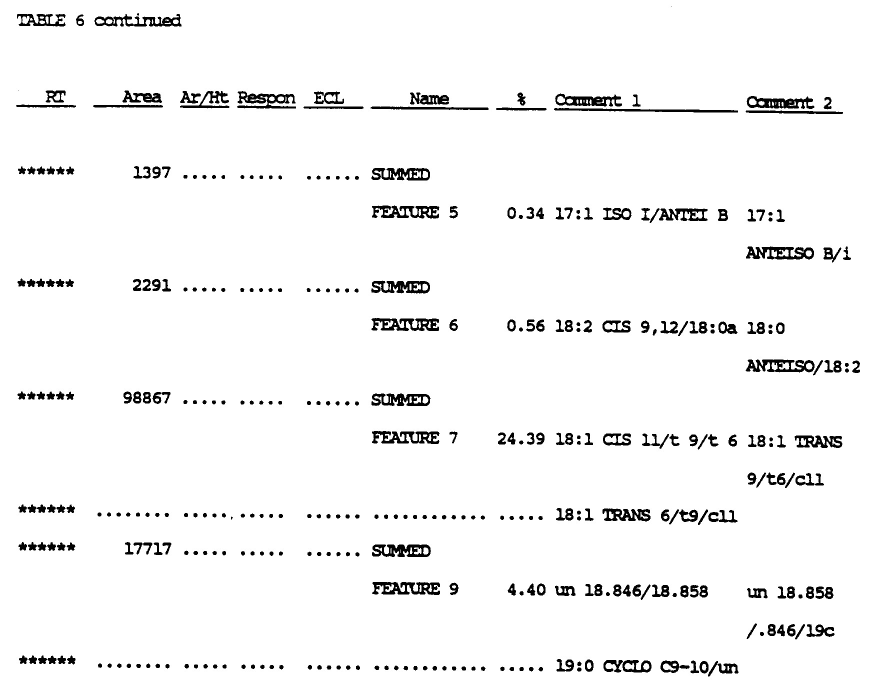

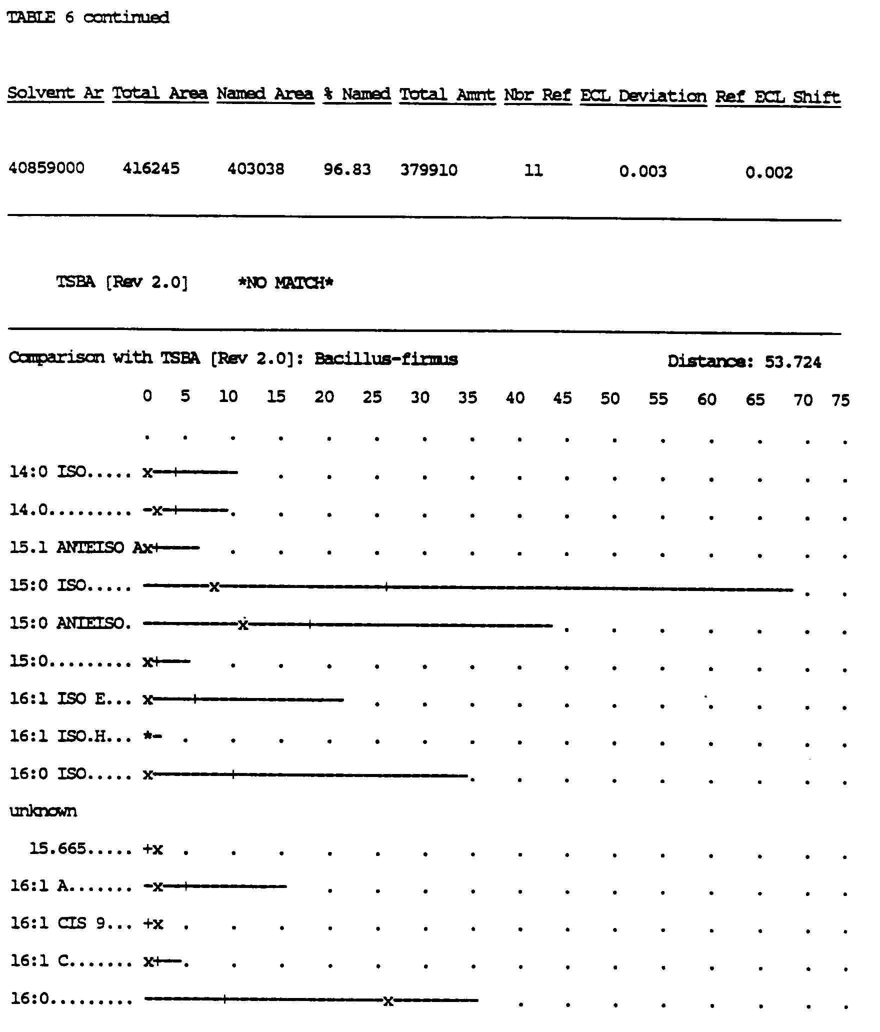

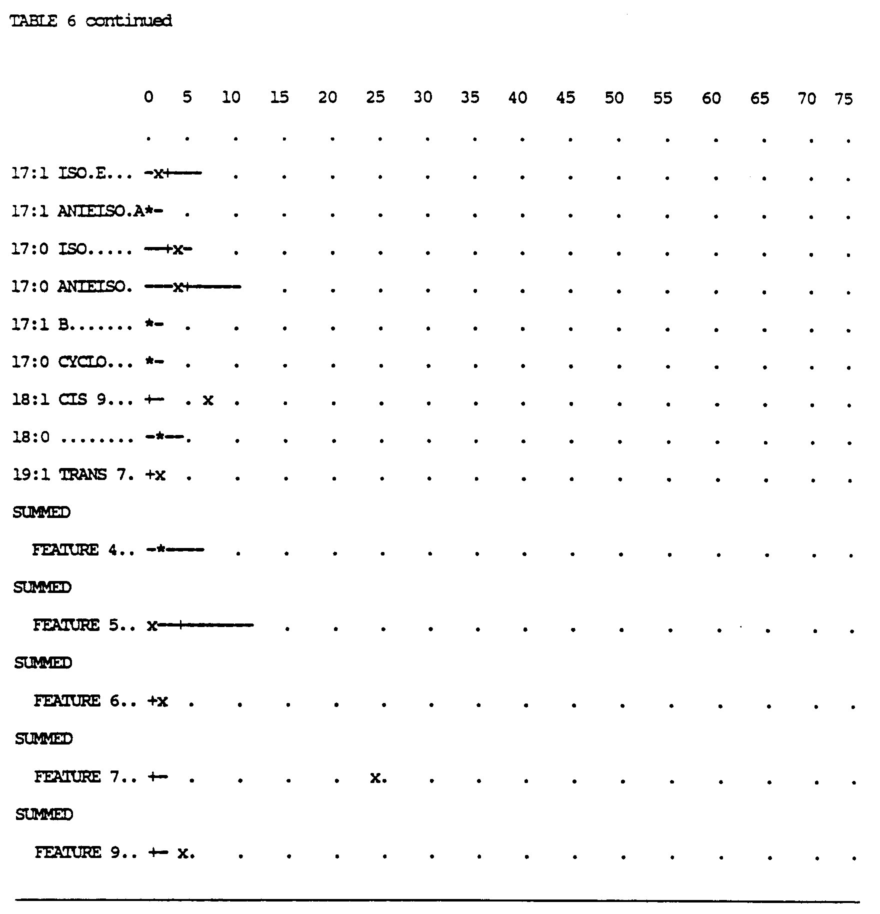

The following non-limitative examples are designed to illustrate the present invention:

### EXAMPLES

### Example 1

Example 1 was conducted to isolate and determine particular bacterial strains which are effective biocontrol agents for the Aphanomyces fungus. Approximately 200 bacterial strains were isolated from pea roots grown in Wisconsin soils infested with Aphanomyces. Each isolate was grown in a nutrient broth (NBY) and coated onto a captan-treated pea seed (Perfection 8221). The term "captan" refers to a fungicide having the chemical name N-(Trichloromethylthio) tetrahydrophthalimide. The coated seeds were air-dried prior to planting.

The coated seeds and the control seeds were then planted in 60 cc. cone-shaped containers, as illustrated in Figure 4, containing either pasteurized soil or naturally infested (with Aphanomyces) field soil. Unless otherwise defined, the control in each of the experiments was a captan-treated pea seed. The pasteurized soil was inoculated with 2 x 10⁴ Aphanomyces zoospores six days after planting. The plants were then grown under greenhouse conditions for approximately three weeks, after which the disease symptoms and shoot dry weights were measured.

The following bacterial strains, listed in Table 7, were identified as the best strains in terms of improvement in shoot dry weights and decreased disease symptoms over control conditions:

**TABLE 7**

| Bacterial Strain | % Shoot Wt. Increase Compared to Control |
|---|---|
| CRK449 | 19.5 |
| 5A | 19.9 |
| CRK424 | 20.0 |
| AMMD | 20.2 |
| PRA44 | 20.2 |
| CRK419 | 20.6 |
| PRA25 | 21.2 |
| PRA42 | 22.6 |
| PRA48 | 23.0 |
| CRK468 | 25.1 |
| CRK478 | 27.7 |
| PRA15 | 45.2 |
| AMMA | 52.7 |

The bacterial strains which showed the greatest promise in reducing pea root rot and disease severity, as well as increasing shoot dry weight, were then tested under field conditions (Examples 2 and 3).

### Example 2

Example 2 was designed to test the twelve bacterial strains, which showed the greatest promise from Example 1, for biocontrol activity. The bacterial strains were cultured and coated onto pea seeds according to the methods described in Example 1. The seeds were then planted in a plot of 17 foot rows of 100 seeds each, each replicated 5 times in a randomized block design. The plants were allowed to grow for one season (8 weeks). Plant mortality was evaluated weekly and the plant yield was determined using the dry weight of the pea plants measured. It is to be noted that the disease was so prevalent in this experiment that no pea pods formed. The results of Example 2 are presented below in Table 8.

**TABLE 8**

| Bacterial Strain | Mean Shoot Dry Wt., g | % Difference** |
|---|---|---|
| control | 61 | -- |
| PRA48 | 36 | -41 |
| PRA44 | 44 | -28 |
| CRK424 | 47 | -23 |
| CRK168 | 61 | + 1 |
| CRK468 | 66 | + 8 |
| PRA42 | 68 | +12 |
| PRA15 | 69 | +22 |
| CRK419 | 79 | +31 |
| PRA25 | 85 | +41 |
| 5A | 92 * | +52 |
| AMMD | 94 * | +55 |
| AMMA | 103 * | +70 |

| | | |
|---|---|---|
| * P less than .05 Dunnett Test | | |
| ** Between the treatments (Bacterial Strain) and the control. | | |

### Example 3

This example, which is similar to Example 2, comprised field trials conducted in locations representing a range of Aphanomyces densities. Example 3 was designed to test five bacterial strains plus a control. The methods and materials were conducted in a manner similar to Example 2. The plant mortality due to Aphanomyces was evaluated weekly. Plant yield was determined using the dry weight of the peas at dry seed stage. The results of this experiment are presented below in Table 9.

**TABLE 9**

| Bacterial Strain | Mean Dry Wt. Peas, g | % Yield Difference |
|---|---|---|
| control | 175 | -- |
| AM | 158 | -10 |
| 5A | 189 | 8 |
| PRA25 | 210 | 12 |
| CRK419 | 215 | 23 |
| AMMD | 282 | 61 |

From Table 3, it can be seen that the bacterial strain AMMD increased the average seed yield by 61%, compared to the non- coated controls.

### Example 4

Like Example 3, Example 4 was designed to test strains of bacteria in the field. Six bacterial strains plus a control were tested under conditions similar to Example 2. Unlike Example 2, the yield here was determined using the fresh weight of peas. The results of Example 4 can be found below in Table 10:

**TABLE 10**

| Bacterial Strain | Mean Fresh Wt. Peas, g | % Yield Difference |
|---|---|---|
| control | 105 | -- |
| UW85 | 119 | 13 |
| CRK419 | 155 | 41 |
| PRA25 | 166 | 58 |
| 5A | 177 | 69 |
| AMMD | 188 | 79 |
| AM | 209 * | 99 |

| | | |
|---|---|---|
| * P less than .05 Dunnett Test. | | |

Several bacterial strains increased pea yield by 13-99%. Pseudomonas cepacia strain AMMD increased yield by 79%, and Pseudomonas fluorescens strain PRA 25 increased yield by 58% compared to the control treatment. It is to be noted that none of the bacterial strains increased the pea yield in fields with less than 1 Aphanomyces propagule per gram of soil.

The next experiments, Examples 5-13, were designed to provide information as to how the bacterial strains work. Although the mechanism of biocontrol by the bacteria is unknown, it has been suggested from tests conducted in petri dishes that the biocontrol bacteria produce a substance which limits the growth of the fungus. This substance may act as an antibiotic in reducing the growth of the fungus in the soil.

### Example 5

Example 5 was conducted to test the effects of the bacterial cultures on the Aphanomyces zoospores. Prior to conducting the test, it was determined that the growth medium, a 1% solution of NBY broth, does not affect the motility of Aphanomyces zoospores. The bacterial strains Pseudomonas cepacia AMMD and Bacillus cereus (UW85) were grown in the NBY growth medium under conditions explained previously with respect to culturing the bacterial strains. The bacterial strains were then diluted to 1% of their original solution and added to a petri dish containing zoospores of the Aphanomyces fungus. The Aphanomyces zoospores were tested for motility after 30 minutes exposure to the bacterial strains, and cyst germination was quantified after 6 hour exposure to the bacterial strains. Cyst germination is a test of the viability of the fungus.

The motility rating scale is as follows:
- 0 =: no motility
- 1 =: a few motile cells
- 2 =: roughly half
- 3 =: most cells motile
- 4 =: full motility as seen at initial release in check treatment.

After 30 minutes exposure to the bacteria and the controls (lake water and 1% NBY broth), the effects on zoospore motility are presented on Table 11:

**TABLE 11**

| Bacterial Strain | Motility |
|---|---|
| lake water * | 3.0 |
| NBY * | 3.0 |
| Bacillus cereus | 1.6 |
| AMMD | 0.2 |

| | |
|---|---|
| * Control | |

Table 12 below illustrates the effect of exposure of the bacterial strains and controls to cyst development in the Aphanomyces fungus after 6 hours:

**TABLE 12**

| Bacterial Strain | % Germlings |
|---|---|
| NBY * | 58.6 |
| AMMD | 22.6 |
| lake water * | 17.4 |
| Bacillus cereus | 13.6 |

| | |
|---|---|
| * Control | |

Replicates of these procedures also demonstrated that AMMA also eliminates zoospore motility in 10 minutes and delays cyst germination.

### Example 6

Example 6 was conducted to compare the effects of certain bacterial strains with a control treatment in zoospore motility of Aphanomyces fungus. The experimental procedure described in Example 5 was followed. The effects on zoospore motility was observed 10 minutes after the bacterial strains (or control) was added to the Aphanomyces treatment. The results are illustrated below in Table 13.

**TABLE 13**

| Treatment * | Motility |
|---|---|
| broth alone | 2.0 |
| AM | 2.0 |
| PRA25 | 1.9 |
| CRK419 | 1.9 |
| BC | 1.8 |
| 5A | 1.4 |
| AMMA | 0.0 |
| AMMD | 0.0 |

| | |
|---|---|
| * Values are means of 5 replicates. | |

### Example 7

Example 7 was conducted to compare the effects of different bacterial strains on mycelial growth, zoospore motility and cyst germination of Aphanomyces. The experimental procedure of Example 5 was followed with respect to Example 7. The results of Example 7 are illustrated below in Table 14.

**TABLE 14**

| Bacterial Strain | Mycelial Growth | Zoospore Motility | Cyst Germination |
|---|---|---|---|
| AMMA | +++* | +++ | +++ |
| AMMD | +++ | +++ | +++ |
| PRA25 | +++ | - | - |
| AM | +++ | - | - |
| CRK419 | -** | - | - |
| 5A | - | + | - |
| UW85 | +*** | - | - |

| | | | |
|---|---|---|---|
| * cessation of activity | | | |
| ** no change in activity | | | |
| *** slight decrease in activity | | | |

### Example 8

Example 8 was conducted to compare the effects of the bacterial strain Pseudomonas cepacia AMMD with a control (NBY broth) on cyst germination. The procedure of Example 5 was used with respect to Example 8. The results of this experiment are illustrated below in Table 15.

**Table 15**

| Treatment | % Cyst Germination |
|---|---|
| broth alone | 58.6 |
| AMMD | 22.6 |

### Example 9

Example 9 was designed to test the in vitro effects of Pseudomonas cepacia AMMD on Aphanomyces zoospores. The Pseudomonas cepacia AMMD bacterial strain was compared to three controls: (1) lake water; (2) cell-free filtrate from NBY-AMMD culture: and (3) NBY growth broth alone. All solutions were diluted to 1% of original in milli-Q water. The motility of the zoospores was rated according to the table illustrated in Figure 5. Table 16 below illustrates the mean of 5 repetitions at 10 and 30 minutes from the following treatments:
- Treatment 1 -: lake water control
- Treatment 2 -: AMMD in NBY broth
- Treatment 3 -: Cell-free filtrate from NBY-AMMD culture
- Treatment 4 -: NBY broth alone

**TABLE 16**

| Time | Treatment: | 1 | 2 | 3 | 4 |
|---|---|---|---|---|---|
| 10 min. | | 3.4 | 0 | 3.6 | 2.6 |
| 30 min. | | 3.8 | 0 | 3.4 | 2.8 |

As illustrated in Table 16, Pseudomonas cepacia AMMD in the NBY broth eliminates zoospore motility after 10 minutes. However, the NBY broth alone and the cell-free cultures of AMMD do not effect zoospore motility.

### Example 10

Example 10 was conducted to test the effects of sugar beet seeds coated with certain bacterial strains on Aphanomyces cochlioides zoospores. Sugar beet seeds were coated with the bacterial strains and planted in a growth chamber under conditions similar to Example 1. The soils were inoculated 6 weeks later with 20 ml. of 103 zoospores per milliliter of Aphanomyces cochlioides. Approximately 6 weeks later, the plants were harvested and the shoots dried and weighed. The results of the shoot dry weight are illustrated below in Table 17.

**TABLE 17**

| Treatment | Shoot Dry Wt. (mg)¹ |
|---|---|
| Control | 5330 |
| BC | 4804 |
| 5A | 5716 |
| AMMD | 9199 * |
| AM | 11533 * |
| AMMA | 11742 * |

| | |
|---|---|
| ¹ Values are means of 15 replicates per treatment. Values marked with an asterisk are significantly different than the controls (Dunnett's test p = 0.05). | |

The bacterial strains Pseudomonas cepacia AMMA, AM, and AMMD significantly increased sugar beet shoot dry weight compared to the control treatment without bacteria.

### Example 11

Example 11 was conducted to compare the effects of various bacterial strains with or without the addition of captan on the emergence of pea shoots. The tests were conducted in soil naturally infested with Aphanomyces euteiches. Pea seeds were coated with the bacteria using the same procedure as for Example 1. In one experiment, treated seeds were planted into flats of soil in the greenhouse. The next experiment was conducted in the field using only three of the bacteria. In both cases, seeds without bacteria served as the check treatments. The results of Example 11 are shown below in Table 18.

**TABLE 18**

| Effects of Bacteria On Pea Emergence. | | | | | | | |
|---|---|---|---|---|---|---|---|
| | % Emergence | | | | | | |
| | | | | | | | |

| Greenhouse Experiment Bacterial Treatment: | | | | | | | |
|---|---|---|---|---|---|---|---|
| | None | 5A | PRA25 | AMMA | AM | CRK419 | AMMD |
| with captan | 84 e | 79 e | 88 de | 92 e | 85 e | 81 e | 62 c |
| without captan | 13 a | 29 ab | 63 cd | 61 c | 31 b | 25 ab | ---- |

| Field Experiment Bacterial Treatment: | | | | | | | |
|---|---|---|---|---|---|---|---|
| | None | | PRA25 | | AM | | AMMD |
| with captan | 88 d | | 92 d | | 88 d | | 89 d |
| without captan | 40 a | | 56 b | | 56 b | | 72 c |

Treatments within each experiment that are not followed by the same letter are significantly different at the P=0.05 level using the Least Significant Difference test.

As illustrated in Table 18, the bacteria strain Pseudomonas cepacia AMMA and Pseudomonas fluorescens PRA25 significantly improved pea emergence from seeds not treated with captan in the greenhouse experiment. Comparing seeds without captan in the field experiment, Pseudomonas cepacia AMMD and Pseudomonas fluorescens PRA25 significantly increased the emergence of peas compared to those without bacteria. None of the bacteria tested improved the emergence of peas treated with captan.

## Claims

1. A process for controlling *Aphanomyces* fungal disease of plants comprising inoculating the plants with a biologically pure culture of a bacterial strain selected from the group consisting of *Pseudomonas cepacia* AMMA (ATCC Accession No. 53796), *Pseudomonas cepacia* AMMD (ATCC Accession No. 53795), *Pseudomonas fluorescens,* (ATCC Accession No. 53794), *Corynebacterium flaccufaciens* 5A (ATCC Accession No. 53934), *Bacillus* strain AM (ATCC Accession No. 53933) and *Bacillus* strain CRK419 (ATCC Accession No. 53935), and mixtures thereof.

2. A process according to claim 1 for controlling *Aphanomyces euteiches* fungal disease.

3. A process according to claim 1 or 2 wherein the plants are pea plants.

4. A process according to claim 1, 2 or 3 wherein the bacterial strain is diluted in a carrier to no less than effective concentration.

5. A process according to any one of the preceding claims which increases germination, decreases mortality and increases yield of a pea plant, which comprising inoculating the pea plant with a growth promotional effective amount of a biologically pure culture of a bacterial strain selected from the group consisting of *Pseudomonas cepacia* AMMA, *Pseudomonas cepacia* AMMD *Pseudomonas fluorescens* PRA25 and mixtures thereof.

6. A biological inoculant for plants suitable for control of *Aphanomyces* fungal disease, comprising a biologically pure culture of *Pseudomonas cepacia* AMMA (ATCC Accession No. 53796), *Pseudomonas cepacia* AMMD (ATCC Accession No. 53795), *Pseudomonas fluorescens* PRA25 (ATCC Accession No. 53794), *Corynebacterium flaccufaciens* 5A (ATCC Accession No. 53934), *Bacillus* strain AM (ATCC Accession No. 53933), or *Bacillus* strain CRK419 (ATCC Accession No. 53935) or a mixture thereof.

7. A biological inoculant according to claim 6 wherein the bacterial strain is diluted in a carrier to no less than effective concentration.

8. An agriculturally useful composition comprising a pea seed inoculated with an inoculant as claimed in claim 6 or 7 comprising *Pseudomonas cepacia* strain AMMA or AMMD.

9. A biologically pure culture of *Pseudomonas cepacia* AMMA (ATCC Accession No. 53796), *Pseudomonas cepacia* AMMD (ATCC Accession No. 53795), *Pseudomonas fluorescens* PRA25 (ATCC Accession No. 53794), *Corynebacterium flaccufaciens* 5A (ATCC Accession No. 53934), *Bacillus* strain AM (ATCC Accession No. 53933) or *Bacillus* strain CRK419 (ATCC Accession No. 53935) suitable for use in the control of *Aphanomyces* fungal disease of plants.

## Patentansprüche

1. Verfahren zur Bekämpfung von Aphanomyces-Pilzerkrankung von Pflanzen, umfassend das Beimpfen der Pflanzen mit einer biologisch reinen Kultur eines Bakterienstammes ausgewählt aus der Gruppe bestehend aus Pseudomonas cepacia AMMA (ATCC Accession No. 53796), Pseudomonas cepacia AMMD (ATCC Accession No. 53795), Pseudomonas fluorescens (ATCC Accession No. 53794), Corynebacterium flaccufaciens 5A (ATCC Accession No. 53934), Bacillus-Stamm AM (ATCC Accession No. 53933) und Bacillus-Stamm CRK419 (ATCC Accession No. 53935) und Mischungen davon.

2. Verfahren nach Anspruch 1, dadurch gekennzeichnet, daß man Aphanomyces euteiches-Pilzerkrankung bekämpft.

3. Verfahren nach Anspruch 1 oder 2, dadurch gekennzeichnet, daß die Pflanzen Hülsenfrüchte sind.

4. Verfahren nach Anspruch 1, 2 oder 3, dadurch gekennzeichnet, daß der Bakterienstamm in einem Träger auf eine Konzentration verdünnt ist, die nicht geringer als die wirksame Konzentration ist.

5. Verfahren nach einem der vorhergehenden Ansprüche, welches die Keimbildung erhöht, die Mortalität verringert und die Ausbeute an Hülsenfrüchten erhöht, dadurch gekennzeichnet, daß man die Hülsenfrüchte mit einer das Wachstum fördenden wirksamen Menge einer biologisch reinen Kultur eines Bakterienstammes beimpft, der ausgewählt ist aus der Gruppe bestehend aus Pseudomonas cepacia AMMA, Pseudomonas cepacia AMMD, Pseudomonas fluorescens PRA25 und Mischungen davon.

6. Biologischer Impfstoff für Pflanzen, der zur Kontrolle von Aphanomyces-Pilzerkrankung geeignet ist, dadurch gekennzeichnet, daß er eine biologisch reine Kultur von Pseudomonas cepacia AMMA (ATCC Accession No. 53796), Pseudomonas cepacia AMMD (ATCC Accession No. 53795), Pseudomonas fluorescens PRA25 (ATCC Accession No. 53794), Corynebacterium flaccufaciens 5A (ATCC Accession No. 53934), Bacillus-Stamm AM (ATCC Accession No. 53933) oder Bacillus-Stamm CRK419 (ATCC Accession No. 53935), oder eine Mischung davon, umfaßt.

7. Biologischer Impfstoff nach Anspruch 6, dadurch gekennzeichnet, daß der Bakterienstamm in einem Träger auf eine Konzentration verdünnt ist, die nicht geringer als die wirksame Konzentration ist.

8. Zusammensetzung zur Verwendung in der Landwirtschaft, dadurch gekennzeichnet, daß sie einen mit einem Impfstoff nach einem der Ansprüche 6 oder 7, der einen Pseudomonas cepacia Stamm AMMA oder AMMD umfaßt, beimpften Hülsenfrüchtesamen umfaßt.

9. Biologisch reine Kultur von Pseudomonas cepacia AMMA (ATCC Accession No. 53796), Pseudomonas cepacia AMMD (ATCC Accession No. 53795), Pseudomonas fluorescens PRA25 (ATCC Accession No. 53794), Corynebacterium flaccufaciens 5A (ATCC Accession No. 53934), Bacillus-Stamm AM (ATCC Accession No. 53933) oder Bacillus-Stamm CRK419 (ATCC Accession No. 53935), zur Verwendung bei der Bekämpfung von Aphanomyces-Pilzerkrankung von Pflanzen.

## Revendications

1. Procédé pour limiter l'infection de plantes par des champignons *Aphanomyces,* lequel procédé comprend l'inoculation des plantes avec une culture biologiquement pure d'une souche bactérienne choisie dans le groupe formé par *Pseudomonas cepacia* AMMA (n° d'enregistrement de l'ATCC 53796), *Pseudomonas cepacia* AMMD (n° d'enregistrement de l'ATCC 53795), *Pseudomonas fluorescents* (n° d'enregistrement de l'ATCC 53794), *Corynebacterium flaccufaciens* 5A (n° d'enregistrement de l'ATCC 53934), *Bacillus* souche AM (n° d'enregistrement de l'ATCC 53933) et *Bacillus* souche CRK419 (n° d'enregistrement de l'ATCC 53935), et des mélanges de ceux-ci.

2. Procédé conforme à la revendication 1 pour limiter une infection par le champignon *Aphanomyces euteiches.*

3. Procédé conforme à la revendication 1 ou 2 dans lequel les plantes sont des pois.

4. Procédé conforme à la revendication 1, 2 ou 3 dans lequel la souche bactérienne est diluée dans un véhicule jusqu'à une concentration qui n'est pas inférieure à la concentration efficace.

5. Procédé conforme à une quelconque des revendications précédentes permettant d'augmenter la germination, de réduire la mortalité et d'augmenter le rendemement des pois, lequel procédé comprend l'inoculation des pois avec une culture biologiquement pure d'une souche bactérienne choisie dans le groupe formé par *Pseudomonas cepacia* AMMA, *Pseudomonas cepacia* AMMD, *Pseudomonas fluorescens* PRA25 et des mélanges de ceux-ci, en une quantité favorisant la croissance.

6. Inoculant biologique pour des plantes capable de limiter l'infection par des champignons *Aphanomyces,* lequel inoculant comprend une culture biologiquement pure de *Pseudomonas cepacia* AMMA (n° d'enregistrement de l'ATCC 53796), *Pseudomonas cepacia* AMMD (n° d'enregistrement de l'ATCC 53795), *Pseudomonas fluorescens* (n° d'enregistrement de l'ATCC 53794), *Corynebacterium flaccufaciens* 5A (n° d'enregistrement de l'ATCC 53934), *Bacillus* souche AM (n° d'enregistrement de l'ATCC 53933) et *Bacillus* souche CRK419 (n° d'enregistrement de l'ATCC 53935), et des mélanges de ceux-ci.

7. Inoculant biologique conforme à la revendication 6 dans lequel la souche bactérienne est diluée dans un véhicule jusqu'à une concentration qui n'est pas inférieure à la concentration efficace.

8. Composition utile pour l'agriculture contenant des graines de pois inoculées avec un inoculant conforme à la revendication 6 ou 7 contenant la souche AMMA ou AMMD de *Pseudomonas cepacia.*

9. Culture biologiquement pure de *Pseudomonas cepacia* AMMA (n° d'enregistrement de l'ATCC 53796), *Pseudomonas cepacia* AMMD (n° d'enregistrement de l'ATCC 53795), *Pseudomonas fluorescen*s (n° d'enregistrement de l'ATCC 53794), *Corynebacterium flaccufaciens* 5A (n° d'enregistrement de l'ATCC 53934), *Bacillus* souche AM (n° d'enregistrement de l'ATCC 53933) et *Bacillus* souche CRK419 (n° d'enregistrement de l'ATCC 53935) appropriée pour être utilisée dans la limitation d'infections de plantes par des champignons *Aphanomyces.*
